# DEMANDE DE BREVET EUROPEEN

(11) **EP 1 195 155 A2**
(43) Date de publication de la demande: **10.04.2002**
(21) Numéro de dépôt: 01402541.5
(22) Date de dépôt: 02.10.2001
(51) Int. Cl.: A61K 7/02

(54) **Composition cosmétique comprenant un pigment goniochromatique**

(30) Priorité: 03.10.2000 FR 0012600
(71) Demandeur: L'OREAL, 75008 Paris (FR)
(72) Inventeur: Grimm, Sabine, 92290 Chatenay Malaby (FR); Simon, Jean-Christophe, 75017 Paris (FR)
(74) Mandataire: Doressamy, Clarisse

(57) **Abrégé**

L'invention concerne une nouvelle composition cosmétique, notamment de maquillage, à phase continue lipophile, de préférence non anhydre, comprenant un pigment goniochromatique à structure multicouche interférentielle. Elle concerne également l'utilisation de ladite composition en maquillage.

## Description

La présente invention concerne une nouvelle composition cosmétique, notamment de maquillage, comprenant un pigment goniochromatique à structure multicouche interférentielle. Elle concerne également l'utilisation de ladite composition en maquillage.

Les compositions de maquillage, tels que les poudres libres ou compactées, les fonds de teint, les fards à joues, les fards à paupières ou les rouges à lèvres, sont constitués d'un véhicule approprié et de différents agents de coloration destinés à conférer une certaine couleur aux compositions avant et/ou après leur application sur la peau (y compris les lèvres) et/ou les phanères.

Pour créer des couleurs, on utilise aujourd'hui une gamme d'agents de coloration assez limitée, en particulier des pigments tels que des laques, des pigments minéraux ou des pigments nacrés. Les laques permettent d'obtenir des couleurs vives, mais pour la plupart sont instables à la lumière, à la température et au pH. Certaines présentent également l'inconvénient de tacher la peau de manière disgracieuse après application, par dégorgement du colorant. Les pigments minéraux, en particulier les oxydes minéraux, sont au contraire très stables, mais donnent des couleurs plutôt ternes et pâles. Les pigments nacrés permettent d'obtenir des couleurs variées, mais jamais intenses, à effets irisés mais le plus souvent assez faibles, et surtout l'effet de couleur est principalement visible selon un seul angle donné correspondant à la réflexion spéculaire. Par exemple le brevet WO-A-96/03.962 décrit une composition cosmétique sous forme d'émulsion comprenant une huile siliconée, un pigment dit interférentiel de type plaquette composé d'un support type mica enrobé d'une couche donnée d'oxyde de titane d'épaisseur donnée, et un pigment à base d'oxyde de fer. Une telle composition donne une teinte selon un angle donné, et n'est pas goniochromatique.

Un des objets de la présente invention est de proposer une nouvelle composition cosmétique qui pallie les inconvénients précités. Un autre des objets de la présente invention est de proposer une nouvelle composition cosmétique qui présente des effets, notamment esthétiques, goniochromatiques et de volume. De tels effets sont notamment visibles pour les fonds de teint, le mascara ou les eye liners. En ce qui concerne la peau, de tels effets sont dits de « morphing », c'est-à-dire de sculpture de la peau sur laquelle elle est appliquée, par la possibilité d'alternance de zones plus sombres et de zones plus claires.

L'invention concerne donc une nouvelle composition cosmétique à phase continue lipophile, de préférence non anhydre, comprenant au moins un pigment goniochromatique à structure multicouche interférentielle et un milieu cosmétiquement acceptable contenant une phase continue lipophile.

Une telle composition présente avantageusement un effet goniochromatique, c'est-à-dire qu'elle est susceptible de produire différentes couleurs selon l'incidence de la lumière et l'angle d'observation, et ce principalement par la présence d'un pigment goniochromatique à structure multicouche interférentielle, dont l'effet propre goniochromatique n'est pas masqué au sein de la composition. L'invention permet ainsi d'obtenir un nouvel effet cosmétique, notamment de maquillage : les couleurs apparaissent et disparaissent selon les mouvements de la personne maquillée et la lumière qui l'éclaire. Par couleur, on entend toute couleur du spectre visible. Le maquillage semble ainsi « vivant », créant avantageusement un effet de volume.

La composition cosmétique peut aussi avantageusement présenter un effet soft-focus, c'est-à-dire un effet flou qui camoufle les micro-reliefs de la peau (y compris les lèvres).

Par pigment, on entend des particules insolubles dans le milieu que constitue la composition cosmétique, c'est-à-dire à l'état dispersé ou solide dans une des phases dudit milieu, et servant à la coloration (création ou modification de teintes de couleur) et/ou à l'opacité de ladite composition.

Par pigment goniochromatique à structure multicouche interférentielle, appelé pigment goniochromatique interférentiel selon l'invention, on entend un pigment à structure au moins bicouche, lesdites couches étant telles qu'elles permettent la création d'un effet de couleur par interférences des rayons lumineux, qui diffractent et diffusent différemment selon les couches. Ainsi de tels pigments peuvent présenter des couleurs variant selon l'angle d'observation et l'incidence de la lumière, et peuvent conférer des reflets irisés.

La structure multicouche peut comporter au moins deux couches, chaque couche, indépendamment ou non de la (ou les) autre(s) couche(s), étant réalisée en au moins un matériau choisi dans le groupe constitué par les matériaux suivants :
MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

De préférence, le pigment goniochromatique à structure multicouche interférentielle selon l'invention est choisi dans le groupe constitué par les pigments goniochromatiques commerciaux suivants : Infinite Colors de SHISEIDO, Sicopearl Fantastico de BASF, Colorstream de MERCK, Xirallic de MERCK, Colorglitter de 3M, et Chromaflair de FLEX.

Par suite la structure multicouche peut être essentiellement minérale ou organique. Selon l'épaisseur de chacune des différentes couches, on obtient différentes couleurs.

Les pigments à structure multicouche interférentielle selon l'invention sont notamment ceux décrits dans les documents suivants : US-A-3 438 796, EP-A-227423, US-A-5 135 812, EP-A-170439, EP-A-341002, US-A-4 930 866, US-A- 5 641 719, EP-A-472371, EP-A-395410, EP-A-753545, EP-A-768343, EP-A-571836, EP-A-708154, EP-A-579091, US-A-5 411 586, US-A- 5 364 467, WO-A-97/39066, DE-A-4 225 031, WO 9517479, DE-A-196 14 637, et leurs associations. Ils se présentent sous forme de paillettes, de couleur métallisée.

Par exemple, la structure multicouche interférentielle est choisie dans le groupe constitué par les structures : Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/ mica-oxyde/SiO₂/Fe₂O₃.

En général, la structure est composée d'une alternance de couches de bas indice optique et haut indice optique.

Le pigment selon l'invention peut être incorporé dans une composition cosmétique ou dermatologique à phase continue lipophile, de préférence non anhydre, notamment de maquillage, en une quantité déterminable aisément par l'homme du métier sur la base de ses connaissances générales, et qui peut notamment aller de 0,01 à 50 %, de préférence de 0,5 à 25 %, en poids par rapport au poids total de la composition. Même à concentration élevée, ce pigment ne déstructure pratiquement pas la composition.

La composition de l'invention peut se présenter sous forme d'un produit à appliquer sur la peau (y compris les les lèvres) et/ou les phanères d'êtres humains, en particulier la peau, aussi bien du corps humain que du visage, les cheveux, les cils et les sourcils . Elle contient donc un milieu cosmétiquement acceptable, c'est-à-dire un milieu compatible avec toutes les matières kératiniques telles que la peau aussi bien du corps humain que du visage, les cheveux, les cils et les sourcils.

Selon l'invention, ce milieu peut comprendre ou se présenter notamment sous forme de suspension, dispersion ou solution dans l'eau ou un milieu hydroalcoolique, éventuellement épaissi, voire gélifié ; émulsion huile-dans-eau (H/E), ou multiple (E/H/E), sous forme de crème, de pâte ou même de solide ; gel aqueux ou hydroalcoolique ou mousse hydrophile ; gel émulsionné ; dispersion de vésicules notamment de lipides ioniques ou non ; lotion biphase ou multiphase ; spray. L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition. De préférence, la composition est non anhydre, c'est-à-dire que ce n'est pas une composition essentiellement exempte d'eau.

La composition comprend donc ledit milieu cosmétiquement acceptable qui comprend une phase continue lipophile, de préférence non anhydre, qui peut contenir au moins un corps lipophile choisi dans le groupe constitué par les corps gras liquide à température ambiante (25 °C généralement) et pression atmosphérique (760 mm Hg généralement soit 1,013. 10⁵ Pa), appelés souvent huiles, les solvants organiques non miscibles à l'eau, et les corps gras solides à température ambiante et pression atmosphérique tels que les cires, les gommes et les corps gras pâteux ou solides, et leurs mélanges. Cette phase continue peut représenter de 0,5 à 99,99% en poids par rapport au poids total de la composition.

Lorsque le milieu se présente sous la forme d'une émulsion, la composition selon l'invention peut éventuellement comprendre, en outre, un tensioactif, de préférence en une quantité de 0 à 30%, de préférence de 0,01 à 30 %, en poids par rapport au poids total de la composition.

Selon l'application envisagée, la composition peut comprendre, en outre, au moins un polymère filmogène, par exemple choisi dans le groupe constitué par les polyuréthannes, les polyacryliques, les hybrides polyuréthannes et polyacryliques, les polyesters, la nitrocellulose, les résines hydrocarbonées et/ou siliconées et leurs mélanges. Ceci est notamment le cas lorsqu'on souhaite préparer une composition de type mascara, eye-liner ou composition capillaire de type laque. Le polymère peut être dissous ou dispersé dans le milieu cosmétiquement acceptable et éventuellement associé à au moins un agent de coalescence et/ou au moins un plastifiant.

Le corps gras liquide à température ambiante et à pression atmosphérique, appelé souvent huile, utilisable dans l'invention, est par exemple choisi dans le groupe constitué par : les huiles hydrocarbonées d'origine animale telles que le perhydrosqualène ; les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides capryli-que/caprique, l'huile de jojoba, l'huile de beurre de karité ; les hydrocarbures linéaires ou ramifiés, d'origine minérale ou synthétique tels que les huiles de paraffine et leurs dérivés, la vaseline, les polydécènes, le polyisobutène hydrogéné tel que le Parléam ; l'isododécane ; les esters et les éthers de synthèse notamment d'acides gras comme par exemple l'huile de Purcellin, le myristate d'isopropyle, le palmitate d'éthyl-2hexyle, le stéarate d'octyl-2-dodécyle, l'érucate d'octyl-2-dodécyle, l'isostéarate d'isostéaryle, l'isononanoate d'isononyle ; les esters hydroxylés comme l'isostéaryl lactate, l'octylhydroxystéarate, l'hydroxystéarate d'octyldodécyle, le diisostéarylmalate, le citrate de triisocétyle ; des heptanoates, octanoates, décanoates d'alcools gras ; des esters de polyol comme le dioctanoate de propylène glycol, le diheptanoate de néopentylglycol, le diisononanoate de diéthylèneglycol, et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante et à pression atmosphérique comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

Ces huiles peuvent représenter de 0 à 90 %, de préférence de 0,01 à 85 %, en poids par rapport au poids total de la phase lipohile.

La composition de l'invention peut comprendre, avantageusement une phase grasse solide ou pâteuse, à température ambiante et à pression atmosphérique, contenant au moins un composé choisi dans le groupe constitué par les cires, les corps gras pâteux, les gommes et leurs mélanges. Les cires peuvent être hydrocarbonées, fluorées et/ou siliconées et être d'origine végétale, minérale, animale et/ou synthétique. En particulier, les cires présentent une température de fusion supérieure à environ 25 °C, de préférence supérieure à environ 45 °C, et les corps gras pâteux une température de fusion d'environ 25 °C à environ 45 °C.

La cire utilisable dans la composition de l'invention est par exemple choisie dans le groupe constitué par la cire d'abeilles, la cire de Carnauba, la cire de Candellila, la cire de Jojoba (hydrogénée ou non), la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone ; leurs mélanges.

La gomme utilisable selon l'invention est généralement choisie dans le groupe constitué par les PDMS à haut poids moléculaire, les gommes de cellulose, les polysaccharides et leurs mélanges, et le corps pâteux utilisable selon l'invention est généralement choisi dans le groupe constitué par les composés hydrocarbonés comme les lanolines et leurs dérivés, les PDMS, et leurs mélanges.

La nature et la quantité des corps solides sont fonction des propriétés mécaniques et des textures recherchées. A titre indicatif, la composition peut contenir de 0 à 50 %, de préférence de 0,01 à 40 %, de façon encore plus préférée de 0,1 à 30 %, en poids par rapport au poids total de la composition, d'au moins un composé choisi dans le groupe constitué par les cires, les corps gras pâteux, les gommes et leurs mélanges.

La composition de l'invention peut, en outre, comprendre une phase particulaire additionnelle pouvant être présente à raison de 0 à 50 %, de préférence de 0,01 à 40 %, de façon encore plus préférée de 0,05 à 30 %, en poids par rapport au poids total de la composition, et qui peut comprendre au moins un pigment autre que le pigment goniochromatique interférentiel selon l'invention et/ou au moins une nacre et/ou au moins une charge, utilisés de façon usuelle dans les compositions cosmétiques.

De façon avantageuse, une telle addition de phase particulaire peut permettre de mieux maîtriser le trajet couleur à travers la composition comprenant le pigment goniochromatique à structure multicouche interférentielle, de façon à éviter certaines teintes qui peuvent apparaître à certains utilisateurs comme inesthétiques (tel que le vert par exemple pour des utilisateurs de fond de teint dans des conditions classiques), ou de façon à augmenter la gamme des teintes proposées aux utilisateurs à partir d'un même pigment goniochromatique à structure multicouche interférentielle. Avantageusement, une telle addition de phase particulaire permet de diminuer le coût de fabrication de la composition cosmétique selon l'invention, car à l'heure actuelle le coût des pigments goniochromatiques est élevé.

Par pigments autres que le pigment goniochromatique interférentiel selon l'invention, il faut comprendre des particules blanches ou colorées, minérales ou organiques, insolubles dans la phase hydrophile liquide, destinées à colorer et/ou opacifier la composition. Le plus souvent de tels pigments sont monocolores. Par charges, il faut comprendre des particules incolores ou blanches, minérales ou de synthèse, lamellaires ou non lamellaires. Par nacres, il faut comprendre des particules irisées, notamment produites par certains mollusques dans leur coquille ou bien synthétisées. Ces charges et nacres servent notamment à modifier la texture de la composition.

Les pigments autres que le pigment goniochromatique interférentiel selon l'invention peuvent être présents dans la composition à raison de 0 à 5 %, de préférence de 0,01 à 5 %, en poids par rapport au poids total de la composition. Au-delà de 5 % en poids, leur présence peut masquer l'effet du pigment goniochromatique interférentiel selon l'invention, ce qui n'est pas acceptable. Ledit pigment est choisi dans le groupe constitué par les exemples cités ci-après. Comme pigments minéraux utilisables selon l'invention, on peut citer les oxydes de titane, de zirconium ou de cérium ainsi que les oxydes de zinc, de fer ou de chrome, le bleu ferrique, et leurs mélanges. Parmi les pigments organiques utilisables selon l'invention, on peut citer le noir de carbone, et les laques de baryum, strontium, zirconium, calcium, aluminium, les dicéto pyrrolopyrrole (DPP) décrits dans les documents EP-A-542669, EP-A-787730, EP-A-787731 et WO 96/08537, et leurs mélanges.

Les nacres peuvent être présentes dans la composition à raison de 0 à 20 %, de préférence de 0,01 à 15% , en poids par rapport au poids total de la composition. Par exemple, la nacre utilisable dans l'invention est choisie dans le groupe constitué par le mica recouvert d'oxyde de titane, d'oxyde de fer, de pigment naturel, d'oxychlorure de bismuth tel que le mica titane coloré, et leurs mélanges.

Les charges peuvent être présentes à raison de 0 à 50 %, de préférence de 0,05 à 30 %, en poids par rapport au poids total de la composition. Par exemple, la charge utilisable selon la présente invention peut être choisie dans le groupe constitué par le talc, le stéarate de zinc, le mica, le kaolin, les poudres de Nylon (Orgasol notamment) et de polyéthylène, le Téflon, l'amidon, le nitrure de bore, les microsphères de copolymères telles que l'Expancel (Nobel Industrie), le Polytrap (Dow Corning), les microsphères de polyméthylmétacrylate, les microbilles de résine de silicone (Tospearl de Toshiba, par exemple), et leurs mélanges.

La composition peut contenir une phase aqueuse, alcoolique ou hydroalcoolique, sous forme dispersée ou émulsionnée dans la phase continue lipophile. Ladite phase lipophile peut donc contenir au moins un corps choisi dans le groupe constitué par l'eau, les alcools, les mélanges d'eau et d'alcool ou d'acétone. De préférence, ledit corps est un alcool notamment choisi dans le groupe constitué par les monoalcools inférieurs linéaires ou ramifiés ayant de 2 à 5 atomes de carbone, comme l'éthanol ou le propanol, les polyols comme la glycérine, la diglycérine, le propylène glycol, le sorbitol, le panthénol, le pentylène glycol, les polyéthylène glycols, et leurs mélanges. Ledit corps peut représenter de 0 à 70 %, de préférence de 0,05 à 60 %, en poids par rapport au poids total de la composition. Ledit corps peut, en outre, contenir au moins un éther en C₂ et au moins un aldéhyde en C₂-C₄ hydrophiles.

La composition selon l'invention peut avoir l'aspect d'une crème, pommade, lotion fluide, de pâte souple de viscosité dynamique à 25 °C de l'ordre de 1 à 40 Pa.s, onguent, solide coulé ou moulé et notamment en stick ou en coupelle.

La composition selon l'invention peut, de plus, comprendre au moins un des ingrédients classiquement utilisés dans les domaines concernés et plus spécialement dans les domaines cosmétique et dermatologique. Ces ingrédients sont en particulier choisis parmi les conservateurs, les épaississants de phase aqueuse (biopolymères polysaccharidiques, les polymères synthétiques) ou grasse, les parfums, les actifs hydrophiles (hydratants , par exemple choisis dans le groupe constitué par l'eau ou les alcools polyhydriques ayant de 2 à 8 atomes de carbone et de 2 à 6 fonctions hydroxy, par exemple choisis parmi l'éthylène glycol, la glycérine, le propanediol-1,2, la diglycérine, l'érythritol, l'arabitol, l'adonitol, le sorbitol, le dulcitol et le D-panthénol) ou lipophiles (par exemple choisi dans le groupe constitué par la lanoline et les filtres UV A ou B), les antioxydants, les colorants, les huiles essentielles, les extraits végétaux, les vitamines et leurs dérivés (telles que les vitamines A, B, C et E), les sphingolipides (céramides), les polymères liposolubles notamment hydrocarbonés (tels que le polybutène, les polyalkylènes, les polyacrylates et les polymères siliconés compatibles avec les corps gras), et leurs mélanges. Les quantités de ces différents ingrédients sont celles classiquement utilisées dans les domaines concernés et par exemple de 0 à 20 %, de préférence de 0,01 à 15 %, en poids par rapport au poids total de la composition. La nature de ces ingrédients et leur proportion doit être compatibles avec l'obtention de compositions à propriétés goniochromatiques selon l'invention.

La composition selon l'invention peut être avantageusement utilisée pour le maquillage de la peau (y compris les lèvres) et/ou des phanères, c'est-à-dire ici de la peau, y compris le lèvres, du visage et du corps humain, des cheveux, des cils ou des sourcils, selon la nature des constituants utilisés. En particulier, cette composition peut être une laque à lèvres, un brillant à lèvres (« gloss » en terminologie anglo-saxonne) utilisable tel quel ou pour appliquer sur un film de rouge à lèvres notamment pour en augmenter son effet de volume (« pulping » en terminologie anglo-saxonne). Elle peut aussi constituer un fond de teint fluide ou solide, un produit anti-cernes ou contours des yeux, un eye liner, un mascara, une ombre à paupières. De façon plus spécifique, l'invention à pour objet un produit à lèvres, un fond de teint ou un mascara.

L'invention a encore pour objet une utilisation cosmétique de la composition ci-dessus pour soigner et/ou maquiller et/ou protéger la peau (y compris les lèvres) et/ou les phanères d'êtres humains, c'est-à-dire ici la peau, y compris les lèvres, du visage et du corps humain, les cheveux, les cils ou les sourcils, ainsi qu'une utilisation de cette composition pour la préparation d'un onguent destiné à traiter et/ou protéger la peau (y compris les lèvres) et/ou les phanères, c'est-à-dire ici la peau, y compris les lèvres, du visage et du corps humain, les cheveux, les cils ou les sourcils. L'invention a aussi pour objet un procédé de traitement cosmétique de la peau, des cheveux, des cils et des sourcils, consistant à appliquer sur la peau (y compris les lèvres) et/ou les phanères, c'est-à-dire ici la peau, y compris les lèvres, du visage et du corps humain, les cheveux, les cils ou les sourcils, la composition selon l'invention.

La composition de l'invention peut être obtenue selon les procédés de préparation classiquement utilisés en cosmétique ou en dermatologie.

L'invention a encore pour objet l'utilisation, dans une composition cosmétique ou pour la fabrication d'une composition dermatologique, colorée, d'un pigment goniochromatique interférentiel compris dans la composition selon l'invention, afin de donner un effet de volume.

L'invention a encore pour objet un procédé de maquillage de la peau (y compris les lèvres) et/ou les phanères, c'est-à-dire ici la peau, y compris les lèvres, du visage et du corps humain, les cheveux, les cils ou les sourcils, consistant à appliquer sur la peau, les cheveux, les cils et les sourcils la composition selon l'invention.

Les exemples de compositions ci-après sont donnés à titre illustratif de l'invention et sans caractère limitatif.

### Exemple 1 : fond de teint anhydre

On a préparé un fond de teint sous forme de stick anhydre ayant la composition suivante :
- isononanoate d'isononyle 15 %
- palmitate d'éthyl-2 hexyle qsp 100 %
- huile de silicone (polydiméthylsiloxane) 17 %
- cires 6 %
- Pigment goniochromatique interférentiel :
   Sicopearl Fantastico Or de BASF 10 %
- poudre de nylon 16%
- cire de PTFE 7 %
- silicone 8 %

On obtient un fond de teint dont la couleur est le jaune / vert, et qui présente un effet de volume une fois appliqué sur la peau par exemple du visage.

De la même façon, en remplaçant le pigment goniochromatique interférentiel Sicopearl Fantastico Or par un pigment goniochromatique interférentiel Sicopearl Fantastico Rose de BASF, on obtient un fond de teint dont la couleur est le rose / rose brun, et qui présente un effet de volume une fois appliqué sur la peau par exemple du visage.

De la même façon, en remplaçant le pigment goniochromatique interférentiel Sicopearl Fantastico Or ou Rose par un pigment goniochromatique interférentiel Sicopearl Fantastico Ruby de BASF, on obtient un fond de teint dont la couleur est le rouge / orange, et qui présente un effet de volume une fois appliqué sur la peau par exemple du visage.

### Exemple 2 : eye liner

On a préparé un eye-liner ayant la composition suivante :
- Cires 6,9 g
- Pigment goniochromatique interférentiel :
   Sicopearl Fantastico Ruby de BASF 10 g
- Agent épaississant 7 g
- Copolymère PLIOWAY® ULTRA 200 10 g
- Copolymère acétate de vinyle/stéarate d'allyle (65/35)
   (polymère liposoluble) 6 g
- Amidon de riz 0,95 g
- Hydrocarbures paraffiniques et naphténiques légers
   (Shell Solt de SHELL) qsp 100 g

On obtient un eye-liner waterproof, anhydre, de couleur noire avec des reflets jaunes à verts suivant l'angle de la lumière et présentant un effet de volume.

### Exemple 3 : mascara

On a préparé un mascara ayant la composition suivante :
- Cire de paraffine 2 g
- Cire de carnauba 4,2 g
- Cire d'abeille 7,4 g
- Polylaurate de vinyle (Mexomère PP de CHIMEX) 0,66 g
- Copolymère acétate de vinyle/stéarate d'allyle (65/35) 1,96 g
- Copolymère PLIOWAY® ULTRA G 20 de GOODYEAR 10 g
- Polymère en dispersion aqueuse* 2,5 g MA
- Amidon de riz 0,74 g
- Pigment goniochromatique interférentiel :
   Sicopearl Fantastico Ruby de BASF 5 g
- Conservateurs qs
- Isododécane qsp 100 g

* Copolymère acétate de vinyle/acide crotonique/tert-butyl-4-benzoate de vinyle (65/10/25) neutralisé à 65 % par l'amino-2 méthyl-2 propanol-1 et plastifié à 25 % par l'adipate de diisopropyle, préparé selon l'enseignement du document EP-A-655234.

Le mascara, non anhydre, s'applique facilement sur les cils. Lorsque l'on applique la composition selon l'invention directement sur les cils noirs, on obtient un maquillage vert très intense qui devient bleu selon l'angle d'observation.

### Exemple 4 : rouge à lèvres

On a préparé une dispersion solide sous forme de bâton de rouge à lèvres selon la demande de brevet européen EP-A1-0.524.892 ayant la composition suivante (en % poids) :
- Huile de ricin 3 %
- Huile de vaseline 9 %
- Lanoline 15%
- Butylhydroxytoluène (BHT) 0,2 %
- Cire d'abeilles 8,8 %
- Ozokérite 10 %
- Lécithine de soja 4 %
- Glycérine 10 %
- Polyéthylène glycol 300 12 %
- Talc 3 %
- D & C Red 27 5%
- D & C Red 7 6%
   Pigment Sicopearl Fantastico Or de BASF 2 %
- Polybutène (INDOPOL de la Société AMOCO) 12 %
- Parfum qs

On obtient un rouge à lèvres dont la couleur est jaune à verte en fonction de l'angle de la lumière, et qui présente un effet de volume une fois appliqué sur les lèvres.

De la même façon, en remplaçant le pigment goniochromatique interférentiel Sicopearl Fantastico Rose par un pigment goniochromatique interférentiel Sicopearl Fantastico Ruby de BASF, on obtient un rouge à lèvres dont la couleur est rose à rose brun en fonction de l'angle de la lumière, et qui présente un effet de volume une fois appliquée sur les lèvres.

### Exemple 5 : fond de teint E/H

On a préparé un fond de teint de type eau-dans-huile ayant la composition suivante :
- tensio-actif vendu sous la dénomination commerciale "Abil WE 09" par la Société Goldschmidt 6 %
- isostéaryl diglycéryl succinate vendu sous la dénomination commerciale "Imwitor 780 K" par la Société Hüls 2 %
- cyclométhicone 25 %
- isododécane 4,55 %
- pigment goniochromatique interférentiel :
   Sicopearl Fantastico Or de BASF 10 %
- poudre de Nylon 8 %
- copolymère acétate de vinyle/p-tertio-butyl-benzoate de vinyl/acide crotonique en dispersion aqueuse partiellement neutralisé, stabilisé 20 %
- adipate de diisopropyle 1 %
- eau qs 100 %

On obtient un fond de teint dont la couleur est le jaune / vert, et qui présente un effet de volume une fois appliqué sur la peau par exemple du visage.

De la même façon, en remplaçant le pigment goniochromatique interférentiel Sicopearl Fantastico Or par un pigment goniochromatique interférentiel Sicopearl Fantastico Rose de BASF, on obtient un fond de teint dont la couleur est le rose / rose brun, et qui présente un effet de volume une fois appliqué sur la peau par exemple du visage.

De la même façon, en remplaçant le pigment goniochromatique interférentiel Sicopearl Fantastico Or ou Rose par un pigment goniochromatique interférentiel Sicopearl Fantastico Ruby de BASF, on obtient un fond de teint dont la couleur est le rouge / orange, et qui présente un effet de volume une fois appliqué sur la peau par exemple du visage.

## Revendications

1. Composition cosmétique à phase continue lipophile non anhydre comprenant au moins un pigment goniochromatique à structure multicouche interférentielle, et un milieu cosmétiquement acceptable contenant une phase continue lipophile, ladite composition comprenant au moins un corps choisi dans le groupe constitué par l'eau, les alcools, les mélanges d'eau et d'alcool ou d'acétone, ledit corps représentant de 0,05 à 60 %, en poids par rapport au poids total de la composition.

2. Composition de maquillage selon la revendication 1.

3. Composition cosmétique selon l'une des revendications 1 ou 2 telle que la structure multicouche comporte au mois deux couches, chaque couche étant réalisée en au moins un matériau choisi dans le groupe constitué par les matériaux suivants : MgF₂, CeF₃, ZnS, ZnSe, Si, SiO₂, Ge, Te, Fe₂O₃, Pt, Va, Al₂O₃, MgO, Y₂O₃, S₂O₃, SiO, HfO₂, ZrO₂, CeO₂, Nb₂O₅, Ta₂O₅, TiO₂, Ag, Al, Au, Cu, Rb, Ti, Ta, W, Zn, MoS₂, cryolithe, alliages, polymères et leurs associations.

4. Composition cosmétique selon l'une des revendications 1 à 4 telle que la structure multicouche interférentielle est choisie dans le groupe constitué par les structures Al/SiO₂/Al/SiO₂/Al ; Cr/MgF₂/Al/MgF₂/Al ; MoS₂/SiO₂/Al/SiO₂/MoS₂ ; Fe₂O₃/SiO₂/Al/ SiO₂/Fe₂O₃ ; Fe₂O₃/SiO₂/Fe₂O₃/SiO₂/Fe₂O₃ ; MoS₂/SiO₂/mica-oxyde/SiO₂/MoS₂ ; Fe₂O₃/SiO₂/ mica-oxyde/SiO₂/Fe₂O₃.

5. Composition selon l'une des revendications 1 à 4 telle que ledit pigment est incorporé dans ladite composition en une quantité de 0,01 à 50 %, de préférence de 0,5 à 25 %, en poids par rapport au poids total de ladite composition.

6. Composition selon l'une des revendications 1 à 5 se présentant sous la forme d'un produit à appliquer sur la peau, y compris les lèvres, aussi bien du corps humain que du visage, les cheveux, les cils et les sourcils.

7. Composition selon l'une des revendications 1 à 6 telle que ledit milieu comprend ou se présente sous forme de suspension, dispersion ou solution dans l'eau ou un milieu hydroalcoolique ; émulsion huile-dans-eau (H/E), ou multiple (E/H/E); gel aqueux ou hydroalcoolique ou mousse hydrophile ; gel émulsionné ; dispersion de vésicules ; lotion biphase ou multiphase ; spray.

8. Composition selon la revendication 7 telle que ledit milieu comprend ou se présente sous la forme d'une émulsion huile-dans-eau (H/E) ou multiple (E/H/E), et que ladite composition comprend en outre de 0 à 30 %, de préférence de 0,01 à 30 %, en poids de tensio-actif par rapport au poids total de ladite composition.

9. Composition selon l'une des revendications 1 à 8 telle que la phase continue lipophile représente de 0,5 à 99,99 % en poids par rapport au poids total de la composition.

10. Composition selon l'une des revendications 1 à 9 telle que ledit milieu comprend au moins un corps lipophile choisi dans le groupe constitué par les corps gras liquides à température ambiante et pression atmosphérique, les solvants organiques non miscibles à l'eau et les corps gras solides à température ambiante et à pression atmosphérique.

11. Composition selon la revendication 10 telle que ledit corps gras liquide à température ambiante et à pression atmosphérique est choisi dans le groupe constitué par les huiles hydrocarbonées d'origine animale; les huiles hydrocarbonées végétales ; les hydrocarbures linéaires ou ramifiés ; les huiles de paraffine et leurs dérivés ; les esters et les éthers de synthèse ; les esters de polyol ; les alcools gras ayant de 12 à 26 atomes de carbone ; les heptanoates, octanoates, décanoates d'alcools gras ; les huiles fluorées partiellement hydrocarbonées et/ou siliconées ; les huiles siliconées les polyméthylsiloxanes (PDMS) ; leurs mélanges.

12. Composition selon l'une des revendications 10 ou 11 telle que ledit corps liquide à température ambiante et à pression atmosphérique est choisi dans le groupe constitué par le perhydrosqualène, les triglycérides des acides heptanoïque ou octanoïque, les huiles de tournesol, de maïs, de soja, de pépins de raisin, de sésame, d'abricot, de macadamia, de ricin, d'avocat, les triglycérides des acides caprylique/caprique, l'huile de jojoba, l'huile de beurre de karité, la vaseline, les polydécènes, le polyisobutène hydrogéné, l'isododécane, l'huile de Purcellin, le myristate d'isopropyle, le palmitate d'éthyl-2 hexyle, le stéarate d'octyl-2-dodécyle, l'érucate d'octyl-2-dodécyle, l'isostéarate d'isostéaryle, l'isononanoate d'isononyle, l'isostéaryl lactate, l'octylhydroxystéarate, l'hydroxystéarate d'octyldodécyle, le diisostéarylmalate, le citrate de triisocétyle, le dioctanoate de propylène glycol, le diheptanoate de néopentylglycol, le diisononanoate de diéthylèneglycol, les esters du pentaérythritol, l'octyldodécanol, le 2-butyloctanol, le 2-hexyldécanol, le 2-undécylpentadécanol, l'alcool oléique ; les cyclométhicones, les diméthicones, les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges.

13. Composition selon l'une des revendications 10 à 14 tel que ledit corps liquide à température ambiante et à pression atmosphérique représente de 0 à 90 %, de préférence de 0,01 à 85%, en poids par rapport au poids total de la phase lipophile.

14. Composition selon l'une des revendications 10 à 13 telle que ledit corps gras solide à température ambiante et pression atmosphérique est choisi dans le groupe constitué par les cires, les corps gras pâteux, les gommes et leurs mélanges.

15. Composition selon la revendication 14 telle que ladite phase lipophile contient de 0 à 50 %, de préférence de 0,01 à 40 %, de façon encore plus préférée de 0,1 à 30 %, en poids par rapport au poids total de la composition, d'au moins un composé choisi dans le groupe constitué par les cires, les corps gras pâteux, les gommes et leurs mélanges.

16. Composition selon la revendication 15 telle que ledit composé est choisi dans le groupe constitué par la cire d'abeilles, la cire de Carnauba, la cire de Candellila, la paraffine, les cires microcristallines, la cérésine, l'ozokérite, les cires synthétiques, les cires de silicones, les PDMS à haut poids moléculaire, les gommes de cellulose et les polysaccharides.

17. Composition selon l'une des revendication 1 à 16 telle que ladite composition comprend en outre au moins un polymère filmogène.

18. Composition selon l'une des revendications 1 à 17 telle que la composition comprend en outre une phase particulaire additionnelle à raison de 0 à 50 %, de préférence de 0,01 à 40 %, de façon encore plus préférée de 0,05 à 30 %, en poids par rapport au poids total de ladite composition.

19. Composition selon la revendication 18 telle que ladite phase particulaire comprend au moins un pigment autre que le pigment goniochromatique à structure multicouche interférentielle et/ou au moins une nacre et/ou au moins une charge.

20. Composition selon la revendication 19 telle qu'elle comprend 0 à 5 %, de préférence de 0,01 à 5 %, de pigment autre que le pigment goniochromatique à structure multicouche interférentielle, en poids par rapport au poids total de la composition.

21. Composition selon l'une des revendications 19 ou 20 telle que ledit pigment est choisi dans le groupe constitué par les oxydes de titane, de zirconium, de cérium, ainsi que les oxydes de zinc et de chrome, le bleu ferrique, le noir de carbone, les laques de baryum, strontium, zirconium, calcium, aluminium, les dicéto pyrrolopyrrole (DPP) et leurs mélanges.

22. Composition selon l'une des revendications 18 à 21 telle qu'elle comprend de 0 à 20 %, de préférence de 0,01 à 15 %, de nacre en poids par rapport au poids total de la composition.

23. Composition selon la revendication 22 telle que la nacre est choisie dans le groupe constitué par le mica recouvert d'oxyde de titane, d'oxyde de fer, de pigment naturel, d'oxychlorure de bismuth, et leurs mélanges.

24. Composition selon l'une des revendications 18 à 23 telle qu'elle comprend de 0 à 50 %, de préférence de 0,05 à 30 %, de charge en poids par rapport au poids total de la composition.

25. Composition selon la revendication 24 telle que la charge est choisie dans le groupe constitué par le talc, le stéarate de zinc, le mica, le kaolin, les poudres de Nylon et de polyéthylène, le Téflon, l'amidon, le nitrure de bore, les microsphères de copolymères, le Polytrap et les microbilles de résine de silicone.

26. Composition selon l'une des revendications 1 à 25 comprenant au moins un corps choisi dans le groupe constitué par les monoalcools inférieurs linéaires ou ramifiés ayant de 2 à 5 atomes de carbone, les polyols et leurs mélanges.

27. Composition selon la revendication 26 comprenant au moins un corps choisi dans le groupe constitué l'éthanol, le propanol, la glycérine, la diglycérine, le propylène glycol, le sorbitol, le panthénol, le pentylène glycol, les polyéthylène glycols, et leurs mélanges.

28. Composition selon l'une des revendications 26 ou 27 telle que ledit corps contient, en outre, contenir au moins un éther en C₂ et au moins un aldéhyde en C₂-C₄ hydrophiles.

29. Composition selon l'une des revendications 1 à 28 telle qu'elle comprend en outre au moins de 0 à 20 %, de préférence de 0,01 à 15 %, en poids par rapport au poids total de la composition d'au moins un ingrédient supplémentaire.

30. Composition selon la revendication 29 telle que l'ingrédient supplémentaire est choisi dans le groupe constitué par les conservateurs, les épaississants de phase aqueuse ou de phase grasse, les parfums, les actifs hydrophiles ou lipophiles, les antioxydants, les colorants, les huiles essentielles, les extraits végétaux, les vitamines et leurs dérivés, les sphingolipides, les polymères liposolubles et leurs mélanges.

31. Composition selon l'une des revendications 29 ou 30 telle l'ingrédient supplémentaire est choisi dans le groupe constitué par l'éthylène glycol, la glycérine, le propanediol-1,2, la diglycérine, l'érythritol, l'arabitol, l'adonitol, le sorbitol, le dulcitol, le D-panthénol, la lanoline, les filtres UV A ou B, les vitamines A, B, C et E et leurs dérivés, le polybutène, les polyalkylènes, les polyacrylates et les polymères siliconés compatibles avec les corps gras.

32. Composition selon l'une des revendications 1 à 31 choisie dans le groupe constitué par les laques à lèvres, les brillants à lèvres, les fonds de teint fluides ou solides, les produits anti-cernes ou de contour des yeux, les eye liners, les mascaras et les ombres à paupières.

33. Utilisation d'une composition selon l'une des revendications 1 à 32 pour le maquillage de la peau, y compris les lèvres, du visage et du corps humain, des cheveux, des cils ou des sourcils.

34. Utilisation d'une composition selon l'une des revendications 1 à 32 pour soigner et/ou maquiller et/ou protéger la peau, y compris les lèvres, du visage et du corps humain, les cheveux , les cils ou les sourcils.

35. Utilisation d'une composition selon l'une des revendicatios 1 à 32 pour la préparation d'un onguent destiné à traiter et/ou protéger la peau, y compris les lèvres, du visage et du corps humain, les cheveux, les cils ou les sourcils.

36. Procédé de traitement cosmétique de la peau, y compris les lèvres, du visage et du corps humain, des cheveux, des cils ou des sourcils consistant à appliquer sur la peau, y compris les lèvres, le visage et le corps humain, les cheveux, les cils ou les sourcils une composition selon l'une des revendications 1 à 32.

37. Utilisation, dans une composition cosmétique ou pour la fabrication d'une composition dermatologique, colorée, d'un pigment goniochromatique interférentiel compris dans une composition selon l'une des revendications 1 à 32, afin de donner un effet de volume.

38. Procédé de maquillage de la peau, y compris les lèvres, du visage et du corps humain, les cheveux, les cils ou les sourcils, consistant à appliquer sur la peau, les cheveux, les cils et les sourcils une composition selon l'une des revendications 1 à 32.
